# EUROPÄISCHE PATENTSCHRIFT

(11) **EP 1 372 619 B1**
(45) Veröffentlichungstag und Bekanntmachung des Hinweises auf die Patenterteilung: **30.05.2012**
(21) Anmeldenummer: 02748317.1
(22) Anmeldetag: 19.02.2002
(51) Int. Cl.: A61K 9/70, A61K 31/465

(54) **VERFAHREN ZUR HERSTELLUNG EINES HOCHFLEXIBLEN TRANSDERMALEN THERAPEUTISCHEN SYSTEMS MIT NIKOTIN ALS WIRKSTOFF**
PROCESS FOR THE PRODUCTION OF A HIGHLY FLEXIBLE TRANSDERMAL THERAPEUTIC SYSTEM HAVING NICOTINE AS ACTIVE SUBSTANCE
PROCEDÉ DE PRÉPARATION D'UN SYSTEME THERAPEUTIQUE TRANSDERMIQUE HAUTEMENT FLEXIBLE RENFERMANT DE LA NICOTINE EN TANT QUE SUBSTANCE ACTIVE

(30) Priorität: 03.03.2001 DE 10110391
(43) Veröffentlichungstag der Anmeldung: 02.01.2004
(73) Patentinhaber: LTS LOHMANN Therapie-Systeme AG, 56626 Andernach (DE)
(72) Erfinder: BRACHT, Stefan, 07751 Cospeda (DE); WARNUS, Sabine, 56729 Ettringen (DE)
(74) Vertreter: Schweitzer, Klaus
(86) Internationale Anmeldenummer: PCT/EP2002/001728
(87) Internationale Veröffentlichungsnummer: WO 2002/069940

(56) Entgegenhaltungen:
- EP-A- 0 484 543
- EP-B1- 0 033 025
- WO-A2-00/64418
- DE-A- 4 332 094
- DE-A- 19 918 106
- US-A- 5 603 947
- US-A- 6 139 868
- PATENT ABSTRACTS OF JAPAN vol. 011, no. 101 (C-413), 31. März 1987 (1987-03-31) & JP 61 251619 A (NITTO ELECTRIC IND CO LTD), 8. November 1986 (1986-11-08)
- VENKATRAMAN S ET AL: "Skin adhesives and skin adhesion - 1. Transdermal drug delivery systems" BIOMATERIALS, ELSEVIER SCIENCE PUBLISHERS BV., BARKING, GB, Bd. 19, Nr. 13, Juni 1998 (1998-06), Seiten 1119-1136, XP004161374 ISSN: 0142-9612

## Beschreibung

Nikotinhaltige transdermale therapeutische Systeme (TTS) sind sowohl auf dem Markt als auch in vielen Patentschriften beschrieben worden. Beispielsweise seien genannt: EP-A 708 627, EP-A 366 240, EP-A 720 474, US-A 5,364,630, WO 95/24172, WO 94/04109, WO 00/33812, WO 88/01516. Weiterhin wird Bezug genommen auf die heute marktführenden TTS-Produkte Nicotinell^{®} (in USA Habitrol^{®}), Nicorette^{®} (in USA Nicotrol^{®}) sowie Nicoderm^{®}.

Die Entwicklung von Nikotin-TTS wird im wesentlichen von zwei Problemkreisen bestimmt:
1. Nikotin ist recht flüchtig. Dies erschwert solche Herstellverfahren, die Prozeßschritte des Trocknens umfassen, bzw. macht sie unmöglich.
2. Nikotin ist ein starker Weichmacher bzw. ein Lösungsmittel für Polymere und Kunststoffolien, wie sie bei der Herstellung von TTS typischerweise zum Einsatz kommen.

Die Problematik unter 1. hat zur Entwicklung von Verfahren geführt, bei denen das nikotinhaltige Material nicht getrocknet werden muß und Nikotin folglich nicht durch Verdampfung oder Verdunstung entweicht.

Die Problematik unter 2. hat zu vielfältigen Beschreibungen von Polymeren und Kunststoffolien geführt, die gegenüber Nikotin besonders resistent sind. Dies betrifft neben Haftkleberformulierungen auch die Schutz- und Rückschichten von TTS sowie geeignete Primärpackmittel.

Die gefundenen Lösungen dieser Probleme sind bisher alle mit einigen Zugeständnissen hinsichtlich eines komplexen Systemaufbaus sowie insbesondere hinsichtlich der teilweise erheblichen Dicke der TTS verbunden.

Um die Beschichtung und Trocknung zu umgehen, wurden Verfahren zum teil-oder völlflächigen Bedrucken von TTS oder einzelnen Schichten davon beschrieben, bei denen jedoch die Anwesenheit saugfähiger Vliese oder Papiere erforderlich ist. Auch kann Nikotin in flüssiger oder angedickter Form in ein Reservoirsystem zudosiert werden. Solche Systeme lassen sich jedoch nur mit erheblichem Aufwand herstellen und sind häufig dick und sehen wenig ansprechend aus. Weiterhin können Heißschmelzverfahren vorgenommen und so die Verwendung von Lösemitteln vermieden und eine Trocknung überflüssig werden. Damit sind jedoch mitunter erhebliche Einschränkungen bei der Auswahl der Polymere und Hilfsstoffe verbunden. Die weichmachenden Eigenschaften des Nikotins erweisen sich insbesondere für die Verwendung der Acrylathaftkleber als problematisch, da diese bereits in reiner Form eine niedrigere Glasübergangstemperatur besitzen, der sie ihre hohe Spontanklebrigkeit auf der menschlichen Haut verdanken. So kommt es schon bei niedrigen Konzentrationen von Nikotin zu einer kritischen weichen Konsistenz, die sich in Schmieren, Fädenziehen und ganz allgemein zu maschinell und manuell ungenügender Handhabbarkeit der Kleberfilme auswirkt.

Aus diesem Grund wurden in Verbindung mit Acrylathaftklebern bisher fast ausschließlich mehrschichtige, besonders dick aufgebaute Systeme vorgeschlagen, die Nikotin jeweils nur in relativ niedriger Konzentration aufnehmen müssen. Aus der Gruppe der Acrylate können zwar auch höhermolekulare Produkte ausgewählt werden, die aufgrund ihres größeren Molekulargewichtes eine größere Resistenz gegenüber weichmachenden Stoffen wie Nikotin aufweisen; diese sind jedoch nur im Heißschmelzverfahren zu verarbeiten. Solche Acrylate stehen jedoch gerade für pharmazeutische Verwendungszwecke, im Gegensatz zu Haftklebern auf Lösemittelbasis, nur in einer sehr begrenzten Auswahl zur Verfügung. In diesem Zusammenhang wurde bisher vielfach Systeme auf der Basis von anderen, weniger nikotinempfindlichen Polymeren vorgeschlagen, wie Kohlenwasserstoffpolymeren wie Polyisobutylen oder Polybutylen sowie Blockcopolymere des Styrols mit Isopren oder Butadien. Weiterhin wurden Silikonpolymere vorgeschlagen, die jedoch unter den verfügbaren Polymeren mit Abstand am teuersten sind. Schließlich weisen die im Markt befindlichen Systeme bei gleicher Abgabeleistung deutliche Größenunterschiede auf, was auch durch die unterschiedliche Resistenz der Systeme gegenüber der enthaltenen Nikotinmenge bedingt ist.

Generell gilt, daß nikotinresistente Polymere ein relativ hohes Molekulargewicht aufweisen und/oder sich durch eine für Haftkleber relativ hohe Glasübergangstemperatur auszeichnen sollten. Dies trifft unter den für die TTS-Herstellung gebräuchlichen Polymeren auf die mit Lösemitteln zu verarbeitenden Acrylathaftkleber am wenigsten zu.

Zusammenfassend kann gesagt werden, daß die heute im Markt befindlichen Nikotin-TTS im Vergleich zu üblichen Matrixsystemen mit anderen Wirkstoffen (z.B. Estrogen) aus den vorgenannten Gründen ungewöhnlich dick und steif konstruiert sind. Dies verringert den Tragekomfort für den Anwender bei Systemflächen von 20 bis 30 cm² erheblich.

### Erfindung

Die Aufgabe der vorliegenden Erfindung bestand darin, ein dünnes und hochflexibles Nikotin-TTS bereitzustellen, das gegenüber üblichen Nikotin-TTS erheblich verbesserte Trageeigenschaften hat. Weiterhin sollte der Aufbau des Systems möglichst einfach gestaltet und die Herstellung unter Verwendung einer lösemittelhaltigen Beschichtung ermöglicht werden.

Diese Aufgabe wird erfindungsgemäß durch ein transdermales therapeutisches System gelöst, das Nikotin und gegebenenfalls weitere am Zentralnervensystem angreifende Wirk- und/oder Hilfsstoffe enthält und aus einer für das Nikotin und die übrigen Wirk- und Hilfsstoffe und vorzugsweise auch für Wasserdampf undurchlässigen Rückschicht (1), einer unmittelbar an die Rückschicht grenzenden, nikotinhaltigen Matrix-Zwischenschicht (2), einer weiteren nikotinhaltigen Matrix-Schicht (3) und einer wiederablösbaren Schutzfolie (4) besteht und dadurch gekennzeichnet ist, daß alle Matrixschichten aus lösemittelhaltig verarbeitbaren (Meth-)Acrylat-Copolymeren aufgebaut sind, wobei die Dicke dieser Schichten (d. h. nur die Matrix-Schichten zusammen mit der Rückschicht, also ohne die Schutzfolie (4)) insgesamt 250 µm nicht überschreitet. In den erfindungsgemäßen transdermalen therapeutischen Systemen werden also allein lösemittelhaltig verarbeitbare (Meth-)Acrylat-Copolymere verwendet, ohne dass festigende Schichten aus Fremdpolymeren oder Stützkörper wie Papier und Vliesstoff hinzugenommen werden müssen.

Unter dem Begriff (Meth-)Acrylat-Copolymere werden dabei Copolymere aus Acrylaten und/oder Methacrylaten verstanden, also Alkylestern, deren Alkylrest zweckmäßig 1 bis 8, vorzugsweise 1 bis 6, insbesondere 1 bis 4 C-Atome enthält. Solche Copolymere können auch unter zusätzlicher Verwendung von Vinylacetat, Acrylsäure und/oder Methacrylsäure hergestellt werden. Unter der Eigenschaft "lösemittelhaltig verarbeitbar" ist zu verstehen, daß die (Meth-)Acrylat-Copolymere in organischen Lösungsmitteln soweit löslich bzw. quellbar sind, daß sie problemlos ausgegossen werden können. (Meth-)Acrylat-Copolymere, die diese Fähigkeit besitzen sind vorzugsweise solche, die ein Molekulargewicht nicht oberhalb von etwa 400 kDa besitzen. Eine scharfe Grenze kann hier nicht zugeordnet werden, da z. B. durch Zugabe bestimmter Hilfsstoffe die Löslichkeit von (Meth-)Acrylat-Copolymeren beeinflusst werden kann.

Vorteilhaft ist das transdermale therapeutische System mit Nikotin soweit beladen, das es mindestens 1,5 mg Nikotin pro cm² Applikationsfläche enthält. Nach einer vorteilhaften Ausführungsform ist das System so gestaltet, daß innerhalb von 24, vorzugsweise innerhalb von 16 Stunden die Abgabe von 0,7 bis 1,4 mg und vorzugsweise von 1,0 bis 1,4 mg Nikotin pro cm² Haut erfolgt.

Die nikotinhaltige Matrix-Zwischenschicht (2) basiert vorteilhaft auf einem Polymer aus der Gruppe der (Meth-)Acrylat-Copolymeren, vorzugsweise Butylmethacrylat-(2-dimethylaminoethyl-methacrylat)-Methylmethacrylat-Copolymer, insbesondere einem solchem mit dem Molverhältnis 1:2:1, oder auch ein Butylmethacrylat-Methylmethacrylat-Copolymer.

Bei der nikotinhaltigen Matrix-Schicht (3) handelt es sich um die hautseitig haftklebende Schicht (Kleberschicht). Diese ist vorzugsweise auf der Basis von Acrylat-Copolymeren aufgebaut, die noch unveresterte Carboxylgruppen, welche von der Acrylsäure bzw. der Methacrylsäure stammen, enthalten und z. B. eine Säurezahl von 20 bis 100 mg KOH je g Polymer aufweisen und welche ganz oder teilweise durch einen oder mehrere basische Zusatzstoffe neutralisiert sein können. Derartige Copolymere sind besonders weichmacherresistent und haben, wenn sie - vorzugsweise teilweise - neutralisiert sind, sehr gute Freisetzungseigenschaften für das Nikotin. Diese transdermalen therapeutischen Systeme können überraschenderweise dünn ausgestaltet werden und weisen selbst im Vergleich zu ähnlich dünnen Konkurrenzprodukten eine erstaunlich hohe Flexibilität auf. Es ist überraschend, daß es durch die Erfindung erstmals ermöglicht wird, derartig dünne und doch zugleich hochflexible Nikotin-TTS herzustellen, deren Biegefestigkeit nicht mehr als 2 cN x cm² beträgt.

Als Zusatzstoffe zu den Carboxylgruppen enthaltenden Acrylat-Copolymeren kommen zum Beispiel in Betracht Alkalihydroxyde, vorzugsweise Kaliumhydroxyd, oder basische Polymere, vorzugweise das bereits erwähnte Butylmethacrylat-(2-dimethylaminoethyl-methacrylat)-Methylmethacrylat-Copolymer. In einer besonders zweckmäßigen Ausführungsform ist das carboxylgruppenhaltige Acrylat-Copolymer durch Aluminium- oder Titanionen quervernetzt.

In den erfindungsgemäßen TTS ist zweckmäßig auch noch mindestens ein stark wasserbindender Hilfsstoff vorhanden, vorzugsweise ein carboxylgruppenhaltiges Polymer oder sein pharmazeutisch akzeptables Salz. Dabei handelt es sich bevorzugt um einen Hilfsstoff aus der Gruppe der Natrium- oder Calcium-Salze von quervernetzter Carboxymethylcellulose oder quervernetzter Polyacrylsäure, wobei dieser Hilfsstoff zweckmäßig pulverförmig in einer oder mehreren der Matrixschichten dispergiert vorliegt. Der Gehalt an dem wasserbindenden Bestandteil in der Matrix beträgt im allgemeinen 1 bis 10 % (m/m), vorzugsweise 2 bis 4 % (m/m).

Als weitere, am Zentralnervensystem angreifende Wirk- und/oder Hilfsstoffe kommen solche in Betracht, die schon bisher im Stand der Technik in Verbindung mit Nikotin für transdermale therapeutische Systeme beschrieben sind, z. B. stabilitätsfördernde Mittel wie übliche Antioxydantien, vorzugsweise Vitamin E und Ascorbylpalmitat.

Unter Flexibilität ist die Fähigkeit eines Flächengebildes zu verstehen, sich durch Biegung einer unebenen Oberfläche anzupassen. Die Haut ist in praktisch allen Bereichen des Körpers eine unebene Oberfläche, um so mehr als sich ihre Oberflächengestalt durch Körperbewegungen ständig ändert. Methode und Ergebnis von Vergleichsversuchen bezüglich der Flexibilität werden weiter unten beschrieben. Aus diesen ergibt sich auch deutlich, daß die geringe Biegefestigkeit (Steifigkeit) von nicht mehr als 2 cN x cm² nicht nur eine Frage geringer Systemdicke, sondern auch des besonders flexiblen Schichtaufbaues bzw. der Matrixformulierung ist. Dies wird durch die Tatsache begünstigt, daß in den erfindungsgemäßen TTS weder gesonderte Wirkstoffdepots noch Stützkörper oder andere verstärkende Elemente sowie Steuermembranen vorhanden sind.

Diese neuartige Formulierung ist gleichzeitig mit einer gegenüber auf dem Markt befindlichen Produkten mindestens gleichwertigen oder sogar überlegenen Abgabeleistung des Nikotin-TTS pro Fläche und Zeit verbunden. Dies wird durch weitere, ebenfalls weiter unten beschriebene Vergleichsuntersuchungen belegt.

Der Einsatz von Nikotin und ggf. flüssiger Hilfsstoffe bedeutet in der Regel eine hohe Belastung der Matrix-Schichten mit weichmachenden Bestandteilen. Für die haftklebende(n) Matrix-Schicht(en) kommen daher insbesondere weichmacherresistente Formulierungen in Betracht. Dies sind z. B. kationisch vernetzbare Acrylatcopolymere. Besonders geeignet sind teilneutralisierte,

carboxylgruppenhaltige Acrylatcopolymere, wie sie in der DE-A 199 18 106 beschrieben sind.

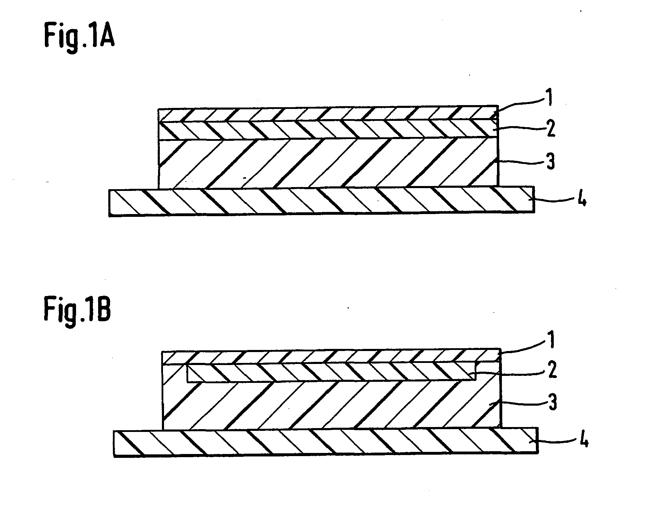

Einen typischen Systemaufbau für ein erfindungsgemäßes Matrix-TTS zeigt Figur 1 A. Auf eine, vorzugsweise wasserdampfundurchlässige Rückschicht (1) folgt eine Matrix-Zwischenschicht (2). Daran anschließend befindet sich eine haftklebende Matrix-Schicht (3). Die haftklebende Oberfläche von (3) ist mit einer Schutzfolie (4) abgedeckt, die vor der Anwendung des TTS entfernt wird.

Abhängig vom Herstellverfahren kann schließlich auch ein Systemaufbau gemäß Figur 1B sinnvoll sein. Die nicht vollflächig ausgeführte Matrix-Zwischenschicht (2) wird z.B. drucktechnisch eingebracht. Die haftklebende Matrix-Schicht (3) steht am Rand in unmittelbarer Verbindung mit der Rückschicht (1) und bildet so einen abschließenden äußeren Rand gegenüber der im Inneren liegenden Matrix-Zwischenschicht (2).

Polymere, die sich zur Verdickung des flüchtigen Bestandteils eignen, wurden in der DE-A 43 32 094 bereits benannt. Solche Polymere sind vorzugsweise nicht haftklebend, da haftklebende Polymere praktisch nicht lösungsmittelfrei im Handel erhältlich und in reiner Form schwer zu verarbeiten sind.

Als besonders geeignet für die vorliegende Erfindung haben sich Polymere auf der Basis von Methacrylsäure oder ihren Estern erwiesen. Typische Vertreter dieser Polymergruppe wie die Eudragit^{®} -Typen, insbesondere Eudragit^{®} E, oder Plastoid^{®} B, beides Produkte der Röhm GmbH, Darmstadt, Deutschland weisen nur mäßige Verdickungswirkung in Lösung auf. Dies ist erwünscht, da auf diese Weisender Polymergehalt der Lösung zu Anfang relativ hoch, im allgemeinen zwischen 20 und 40% (m/m) gehalten werden kann. Im Rahmen der Gleichgewichtseinstellung im weiteren Verlauf kann somit rasch eine Verfestigung der Lösung zu einem kohäsiven Polymerfilm von ausreichender Dicke und Festigkeit einsetzen. Weiterhin zeigen Polymethacrylate gute Verankerungseigenschaften auf bahnförmigen Trägern aus Polyethylenterephthalat (PET), die ihrerseits das bevorzugte Material für Rückschichten von TTS darstellen.

Der Wirkstoffgehalt der eingesetzten Nikotinlösung läßt sich neben dem enthaltenen Polymer durch Zusatz weiterer Hilfsstoffe bedarfsweise auch herabsetzen. Dies kann zur Abstimmung des Nikotineintrages in das System pro Fläche, aber auch zur Einstellung einer geeigneten Viskosität für die Beschichtung mit dieser Lösung sinnvoll sein. Weiterhin können solche Zusätze klebrigkeitsverbessernd wirken und nach der Gleichgewichtseinstellung des Systems die Haftung der Einzelschichten im Verbund erhöhen. Solche Zusatzstoffe können vorzugsweise, jedoch ohne Anspruch auf Vollständigkeit sein: Triglyceride gesättigter Fettsäuren (z.B. Miglyol^{®} 812) (ein gemischt saures Triglycerid der fraktionierten Kokosfettsäuren der Fa. Degussa, Deutschland) Monoglyceride von Fettsäuren (z.B. Glycerinmonolaurat oder Glycerinmonooleat), Ester des Methanols, Ethanols, Isopropanols oder Propylenglykols mit Fettsäuren (z.B. Isopropylpalmitat) sowie Hart- und Weichharze in Form von Derivaten der Abietinsäure.

Als geeignete Materialien für die Rückschicht des TTS sind Folien mit geringer Nikotindurchlässigkeit vorzuziehen. Dies sind beispielsweise Polyethylenterephthalat, z. B. Hostaphan^{®} der Fa. Mitsubishi (PET) oder thermoplastische Acrylnitril-Copolymerisate, wie sie durch Pfropf-Polymerisation von 73-77 Teilen Acrylnitril und 23-27 Teilen Methylacrylat in Gegenwart von 8-18 Teilen Butadien-Acrylnitril-Copolymer mit 70 % Butadien (Teile und Prozente beziehen sich jeweils auf das Gewicht) erhalten und unter der Bezeichnung Barex^{®} (früher: Marke der Fa. Vistron Corp., Cleveland, Ohio, USA; jetzt BP) vertrieben werden. (vgl. M. Th. Schuler in Kunststoffe-Plastics, 9/1974, Seiten 13-20). Diese Folien können aus kosmetischen Gründen lackiert sein. Zum Lichtschutz für die nikotinhaltigen Matrixschichten kann bedarfsweise eine Aluminisierung aufgebracht sein.

### Vergleichsversuche bezüglich der Flexibilität

Um diese vom Verbraucher sehr leicht wahrnehmbare Produktqualität objektivierbar zu machen, wurde in Abwandlung die DIN 53 362-Bestimmungsmethode der Biegefestigkeit (Verfahren nach Cantilever) von Kunststofffolien und textilen Flächengebilden angewendet und hierfür ein der Norm entsprechendes Meßgerät der Fa. Richard Hess MBV GmbH, 47663 Sonsbeck, Deutschland benutzt. Im Prinzip wird hierbei eine streifenförmige Probe des zu prüfenden Produktes über eine Kante frei in den Raum vorgeschoben. Mit zunehmender Überhanglänge biegt sich das flächige Produkt unter dem Einfluß des eigenen Gewichts nach unten. Diejenige Überhanglänge, bei der ein vorgegebener Biegewinkel von 41 °30' erreicht ist, wird notiert und in Abhängigkeit vom Eigengewicht der Probe in einen Meßwert für Biegefestigkeit in der Einheit [cN x cm²] umgerechnet.

Als Probekörper wurden aus fertigen TTS ausgeschnittene Streifen verwendet. Dabei mußte die Länge der Probekörper, abweichend von der Längenvorgabe 250 mm in der DIN-Vorschrift, auf die jeweils aus kommerziellen Produkten verfügbare Länge begrenzt werden (die tatsächlichen Längen der Probekörper lagen zwischen 40 und 70 mm). Von den Probekörpern wurde vor der Messung die Schutzfolie der Kleberschicht entfernt. Die Kleberschicht wurde in Übereinstimmung mit der o.g. DIN-Vorschrift mit Talkum eingepudert, bis sie nicht mehr klebte.

Die Meßergebnisse (Mittelwert aus n=2 Bestimmungen) sowie die zugehörigen Längen der Probekörper sind in Tabelle 1 aufgeführt. Die Biegefestigkeit wurde bei jedem Probekörper in beide Richtungen ermittelt, d.h. in einer ersten Messung mit der Rückschicht nach oben und in einer zweiten Messung mit der Rückschicht nach unten weisend. Dies geschah im Hinblick auf die Tatsache, daß ein TTS beim Tragen auf der Haut typischerweise in beide Richtungen flexibel sein soll. Es wurde ein Nikotin-TTS gemäß Beispiel 1 mit drei im Markt befindlichen Nikotin-TTS verglichen sowie weiterhin mit 2 estradiolhaltigen am Markt erhältlichen Produkten, die hier als Positivreferenz in Bezug auf hohe Flexibilität und hohen Tragekomfort für den Anwender dienten.

**Tab.1:**

| | **Nikotin-TTS A** | **Nikotin-TTS B** | **Nikotin-TTS C** | **Nikotin-TTS nach Beispiel 1** | **Estradiol-TTS E** | **Estradiol-TTS F** |
|---|---|---|---|---|---|---|
| Biegefestigkeit Richtung A | >14,5 cN x cm² | >8,9 cN x cm² | n.a. | 1,4 cN x cm² | 1,16 cN x cm² | 0,94 cN x cm² |
| Biegefestigkeit Richtung B | >14,5 cN x cm² | >8,9 cN x cm² | 3,33 cN x cm² | 1,16 cN x cm² | 0,27 cN x cm² | 0,8 cN x cm² |
| Systemdicke ohne Schutzfolie) | 238 µm | 389 µm | 394 µm | 218 µm | 110 µm | 117 µm |

| | | | | | | |
|---|---|---|---|---|---|---|
| n.a.: nicht anwendbar, wegen Einrollung des Probekörpers | | | | | | |

Nikotin-TTS A bezieht sich auf das Produkt Nicorette®, eine Marke der Pharmacia & Upjohn GmbH, Nikotin-TTS B auf Nicoderm^{®} CQ, eine Marke der SmithKline Beecham Consumer Healthcare L.P. (USA), Nikotin-TTS C auf Nicotine Transdermal USP, das von Schein Pharmaceutical, Inc. (USA) vertrieben wird. Estradiol-TTS E bezieht sich auf Dermestril^{®}, eine Marke der Firma Rottapharm s.r.l.(Monza, Italien), und Estradiol-TTS F auf Fem7®, eine Marke der Merck KGaA, Darmstadt, Deutschland.

Richtung A bedeutet eine Messung des Probekörpers, bei der die TTS-Rückschicht nach unten weist. In Richtung B wurde dagegen mit der Rückschicht oben liegend gemessen.

In einigen Fällen wurde der Biegewinkel von 41 °30' von den Probekörpern in der verfügbaren Länge wegen zu großer Steifigkeit überhaupt nicht erreicht.

In diesen Fällen wurde davon ausgegangen, daß der Meßwert größer sein muß als derjenige Wert, der sich aus Erreichen des kritischen Winkels durch den Probekörper in der hier möglichen Überhanglänge ergeben haben würde.

Im Fall des Nikotin-TTS C wies das Produkt eine starke Neigung zur Einrollung bzw. Kräuselung auf. Dies machte eine Messung in der Richtung A unmöglich. Es konnten nur die Meßwerte in Richtung B herangezogen werden.

Aus diesen Meßwerten wird erkennbar, daß das erfindungsgemäße Nikotin-TTS eine deutlich geringere Biegefestigkeit hat als die verglichenen TTS mit dem Wirkstoff Nikotin. Überraschenderweise liegen die Werte sogar im Bereich zweier einschichtiger, dünner Matrix TTS mit dem Wirkstoff Estradiol. Diese können näherungsweise als flexibelste TTS auf dem jetzigen Markt gelten.

Überraschenderweise liegt die Biegefestigkeit des erfindungsgemäßen Nikotin-TTS auch sehr deutlich unterhalb der des Nikotin-TTS A. Beide Systeme unterscheiden sich in der Dicke nur unwesentlich (Nikotin-TTS A ca. 238 µm, erfindungsgemäßes Nikotin-TTS ca. 218 µm)

### Beispiel 1 (herkömmliches Herstellverfahren)

Aus der DE-A 43 32 094 sind Produkte und Verfahren bekannt, die die Einbringung flüchtiger pharmazeutischer Wirk- oder Hilfsstoffe in TTS ermöglichen. Zu diesem Zweck wird der flüchtige Bestandteil durch Auflösung eines Polymers und gegebenenfalls weiterer fester Hilfsstoffe in dem flüchtigen Bestandteil als Lösungsmittel soweit angedickt, daß diese Lösung auf einen bahnförmigen Träger beschichtet werden kann (= Verfahrensschritt 1). Dieses Beschichtungsprodukt wird dann mit separat hergestellten, weiteren Schichten des TTS laminiert, wonach durch Diffusion eine Gleichgewichtseinstellung des flüchtigen Stoffes im Gesamtsystem stattfinden kann (= Verfahrensschritt 2). Dabei verfestigt sich die zuvor flüssige Schicht durch Diffusion des flüchtigen Bestandteils als einzigem Lösungsmittel in die übrigen Schichten des TTS.

Die Polymerlösung für den Verfahrensschritt 1 weist die Zusammensetzung gemäß. Tabelle 2 auf. Diese wird durch ein Auftragswerk flächig auf eine Folie aus Polyethylenterephthalat der Dicke 19 µm mit einem Flächengewicht von 54 g/m² beschichtet. Das Beschichtungsprodukt wird sofort in Verfahrensschritt 2 mit einer Haftkleberschicht der Zusammensetzung gemäß Tabelle 3 und einer Schichtdicke von 144 g/m² zusammenkaschiert. Das erhaltene Produkt wird 10 Minuten auf 60°C erwärmt und dann auf eine Rolle gewickelt. Dieses Produkt wird sofort oder auch nach Zwischenlagerung in üblicher Weise durch Längsschneiden und Stanzen zu TTS weiterverarbeitet.

**Tab.2:**

| **Bezeichnung** | **Menge [%]** | **Funktion** |
|---|---|---|
| Nikotin | 32,41 | Flüchtiger Wirkstoff |
| Eudragit^{®} EPO | 27,00 | Verdickendes Methacrylat-Copolymer |
| Miglyol^{®} 812 | 40,23 | Hilfsstoff |
| Vitamin E | 0,36 | Antioxidans |

**Tab.3:**

| **Bezeichnung** | **Menge [%]** | **Funktion** |
|---|---|---|
| Durotak® 387-2051 | 95,18 | Acrylatcopolymer-Haftkleber |
| KOH | 0,77 | Neutralisationsreagenz |
| Aluminium* | 0,05 | Vernetzungsreagenz für Durotak |
| Natriumsalz der quervernetzten Carboxymethyl-cellulose | 4,00 | Hydroadsorbens |

| | | |
|---|---|---|
| *Das Aluminium wurde als Al-acetylacetonat zugesetzt. | | |

Die Herstellung der Kleberschicht gemäß Tabelle 3 erfolgt in einem konventionellen, lösemittelhaltigen Beschichtungsverfahren mit anschließender Trocknung. Als Lösemittel wurde eine Gemisch von Ethylacetat, Methanol und Acetylaceton verwendet. Die Beschichtung erfolgte auf eine silikonisierte Schutzfolie aus Polyethylenterephthalat (100 µm Hostaphan^{®} Folie).

Das erhaltene TTS weist einen Wirkstoffgehalt von ca. 1,75 mg Nikotin je cm² Applikationsfläche auf. Dies entspricht recht genau dem Gehalt des Nikotin-TTS D.

Die Dicke des erfindungsgemäßen Systems beträgt jedoch nur ca. 218 µm (ohne Schutzfolie bestimmt) und liegt damit unter der der vorgenannten Systeme.

### Pharmakokinetische Vergleichsuntersuchungen

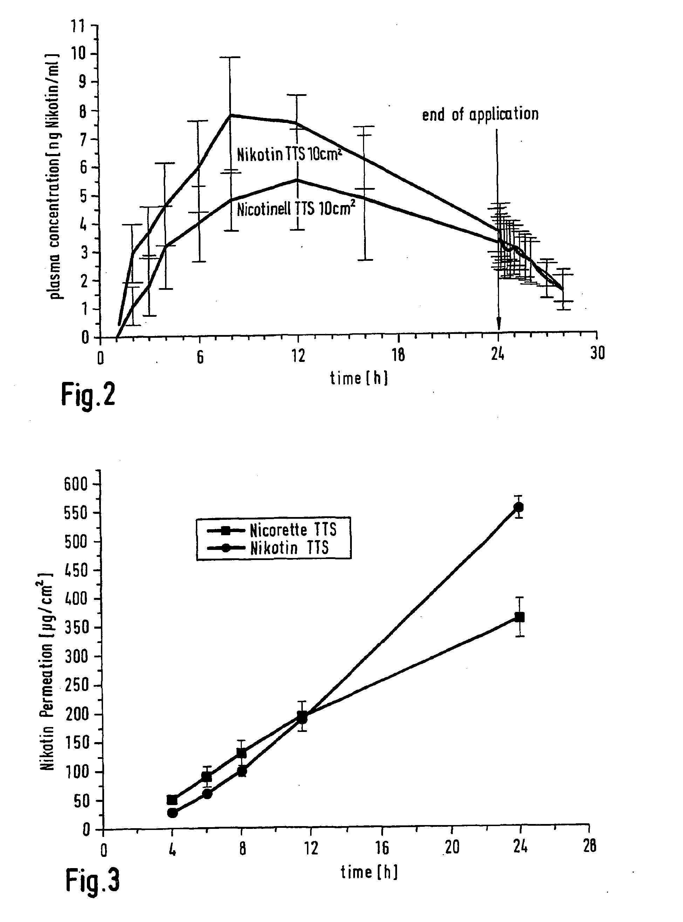

Das erfindungsgemäße Nikotin-TTS des Beispiels 1 wurde in einer pharmakokinetischen Studie am Menschen (6 gesunde, männliche Probanden, die nacheinander das erfindungsgemäße und das Vergleichspräparat anwenden - cross-over Design) mit einem Nikotin-TTS D (Nicotinell^{®} TTS der Novartis Consumer Health Care S. A., Basel Schweiz), verglichen. Die in Figur 2 dargestellten Flächen unter der Plasmaspiegelkurve belegen eine Überlegenheit des erfinderischen Nikotin TTS in der Größenordnung von 140%, bezogen auf flächengleiche TTS. Dies entspricht einer Abgaberate von ca. 1 mg Nikotin pro cm² in 24 Stunden, gegenüber dem bei Nikotin-TTS D deklarierten Wert von 0,7 mg pro cm² in 24 Stunden.

Ein Vergleich wurde auch mit dem Nikotin-TTS A in vitro durchgeführt. Die Permeationen wurden an Humanhaut in dafür gebräuchlichen modifizierten Diffusionszellen nach Franz bestimmt. Die Versuchstemperatur betrug 32°C, als Akzeptormedium wurde ein wäßriger Puffer vom pH 5,5 eingesetzt. Alle Angaben sind Mittelwerte aus n=3 Proben von der Haut desselben Spenders, ermittelt mit TTS-Stanzlingen von 1,12 cm² Fläche. Die in Figur 3 dargestellten Ergebnisse zeigen die Überlegenheit des erfindungsgemäßen Nikotin-TTS gegenüber dem Nikotin-TTS A hinsichtlich der Gesamtabgabemenge von Nikotin pro cm² in 24 Stunden.

### Beispiel 2 (erfindungsgemäßes Verfahren)

Aus EP-B 0 303025 ist ein Druckverfahren bekannt, mit dessen Hilfe Nikotin in das einzelne TTS eingebracht werden kann. Mit Hilfe dieses Prinzips wurde unter Zuhilfenahme eines Druckwerkes der Fa. Tampoprint die Wirkstofflösung gemäß Tabelle 4 direkt auf eine Haftkleberschicht der Zusammensetzung gemäß Tabelle 5 flächig rund aufgedruckt. Dabei wurden ca. 25 mg Nikotinlösung auf eine Kleberfläche von ca. 6 cm² verdruckt. Das Flächengewicht der Haftkleberschicht gemäß Tabelle 5 betrug 144 g/m².

Nach dem Aufdruck wurde die bedruckte Kleberschicht sofort mit einer Rückschicht aus Polyethylenterephthalat (15 µm Hostaphan-Folie) abgedeckt und mechanisch mit dieser zusammenkaschiert. Die bedruckten Flächen wurden aus diesem Verbund mit einem runden Stanzwerkzeug ausgestanzt. Dabei wurde ein Durchmesser des Stanzlinges von etwa 4 mm Überschuß (entsprechend ca. 8 cm² Fläche) gegenüber dem Durchmesser der bedruckten Fläche gewählt. So wurde ein System gemäß Figur 1 B hergestellt. Bei der Lagerung erfolgte eine Gleichgewichtseinstellung des Nikotins innerhalb aller Schichten des TTS.

**Tab.4:**

| **Bezeichnung** | **Menge [%]** | **Funktion** |
|---|---|---|
| Nikotin | 60,00 | Flüchtiger Wirkstoff |
| Eudragit^{®} 100 | 40,00 | Verdickendes Methacrylat-Copolymer |

**Tab.5:**

| **Bezeichnung** | **Menge [%]** | **Funktion** |
|---|---|---|
| Durotak 387-2051 | 85,26 | Acrylatcopolymer-Haftkleber |
| KOH | 0,69 | Neutralisationsreagenz |
| Aluminium* | 0,05 | Vemetzungsreagenz für Durotak |
| Natriumsalz der quervernetzten Carboxylmethyl-cellulose | 4,00 | Hydroadsorbens |
| Eudragit E 100 | 10,00 | basischer Hilfsstoff |

| | | |
|---|---|---|
| *Das Aluminium wurde als Al-acetylacetonat zugesetzt. | | |

Die Herstellung der Kleberschicht gemäß Tabelle 5 erfolgt in einem konventionellen, lösemittelhaltigen Beschichtungserfahren mit anschließender Trocknung. Als Lösemittel wurde ein Gemisch von Ethylacetat, Methanol und Acetylaceton verwendet. Die Beschichtung erfolgte auf eine silikonisierte Schutzfolie aus Polyethylenterephthalat (100 µm Hostaphan^{®}-Folie).

## Patentansprüche

1. Verfahren zur Herstellung eines transdermalen therapeutischen Systems mit einem Gehalt an Nikotin, bestehend aus
- einer für das Nikotin undurchlässigen Rückschicht,
- einer unmittelbar an die Rückschicht grenzenden, nikotinhaltigen Matrix-Zwischenschicht,
- einer weiteren nikotinhaltigen Matrix-Schicht,
- wobei die Matrixschichten aus (Meth-)Acrylat-Copolymeren aufgebaut sind, und
- einer wiederablösbaren Schutzschicht,
- wobei die Dicke dieser Schichten insgesamt 250 µm nicht überschreitet und der Gehalt an Nikotin mindestens 1,5 mg pro cm² Applikationsfläche beträgt,
**dadurch gekennzeichnet, dass**
die nikotinhaltige Matrix-Zwischenschicht drucktechnisch nicht-vollflächig eingebracht wird, so dass die weitere Matrix-Schicht einen abschließenden äußeren Rand gegenüber der im Inneren liegenden nikotinhaltigen Matrix-Zwischenschicht bildet.

2. Verfahren nach Anspruch 1, **dadurch gekennzeichnet, dass** die Biegefestigkeit des auf die Haut zu applizierenden transdermalen Systems nicht mehr als 2 cN·cm² beträgt.

3. Verfahren nach Anspruch 1 oder 2, **dadurch gekennzeichnet, dass** das transdermale System frei von Stützkörpern oder festigenden Schichten ist.

4. Verfahren nach Anspruch 1, 2 oder 3, **dadurch gekennzeichnet, dass** das transdermale System für eine Abgabemenge von 0,7 bis 1,4 mg, vorzugsweise 1,0 bis 1,4 mg Nikotin pro cm² Haut innerhalb von 24 Stunden, vorzugsweise für eine Applikationsdauer von 16 Stunden vorgesehen ist.

5. Verfahren nach einem oder mehreren der vorangehenden Ansprüche, **dadurch gekennzeichnet, dass** die Zwischenschicht auf einem Polymer aus der Gruppe der Methacrylat-Copolymeren basiert, vorzugsweise Butylmethacrylat-(2-Dimethylaminoethyl-methacrylat)-Methylmethacrylat-Copolymer oder ein Butylmethacrylat-Methylmethacrylat-Copolymer ist.

6. Verfahren nach einem oder mehreren der vorangehenden Ansprüche, **dadurch gekennzeichnet, dass** die Matrix-Schicht die hautseitig haftklebende Schicht ist und auf der Basis von Acrylat-Copolymeren aufgebaut ist.

7. Verfahren nach Anspruch 6, **dadurch gekennzeichnet, dass** das haftklebende Acrylat-Copolymer carboxylgruppenhaltig ist, wobei dieses zweckmäßig in einer durch einen oder mehrere basische Zusatzstoffe ganz oder teilweise neutralisierten Form und/oder in durch Aluminium- oder Titan-Ionen quervernetzter Form vorliegt und es sich bei dem basischen Zusatzstoff vorzugsweise um ein Alkalihydroxid, insbesondere Kaliumhydroxid, oder um ein basisches Polymer, Butylmethacrylat-(2-Dimethylaminoethyl-methacrylat)-Methylmethacrylat-Copolymer (1:2:1) handelt.

8. Verfahren nach Anspruch 7, **dadurch gekennzeichnet, dass** in dem transdermalen System mindestens ein stark wasserbindender Hilfsstoff enthalten ist, vorzugsweise ein carboxylgruppenhaltiges Polymer oder sein pharmazeutisch akzeptables Salz.

9. Verfahren nach einem oder mehreren der vorangehenden Ansprüche, **dadurch gekennzeichnet, dass** die die Matrixschichten aufbauenden (Meth-)AcrylatCopolymere lösemittelhaltig verarbeitbar sind.

10. Verfahren nach einem oder mehreren der vorangehenden Ansprüche, **dadurch gekennzeichnet, dass** mindestens ein weiterer, am Zentralnervensystem angreifender Wirk- und/oder Hilfsstoff enthalten ist.

## Claims

1. Process for producing a transdermal therapeutic system with nicotine content, composed of
- a backing layer impermeable to the nicotine,
- a nicotine-containing intermediate matrix layer immediately adjoining the backing layer,
- another nicotine-containing matrix layer,
- where the matrix layers are composed of (meth)acrylate copolymers, and
- a peelable protective layer,
- where the total thickness of the said layers does not exceed 250 µm and the nicotine content is at least 1.5 mg per cm² of application area,
**characterized in that**
the nicotine-containing intermediate matrix layer is introduced by a printing technique in a manner which does not cover the entire surface, so that the other matrix layer provides an outermost edge to the nicotine-containing intermediate matrix layer situated in the interior.

2. Process according to Claim 1, **characterized in that** the flexural strength of the transdermal system to be applied to the skin is not more than 2 cN·cm².

3. Process according to Claim 1 or 2, **characterized in that** the transdermal system is free from supportive structures and from reinforcing layers.

4. Process according to Claim 1, 2 or 3, **characterized in that** the transdermal system is intended to dispense an amount of from 0.7 to 1.4 mg, preferably from 1.0 to 1.4 mg of nicotine per cm² of skin within a period of 24 hours, preferably for an application time of 16 hours.

5. Process according to one or more of the preceding claims, **characterized in that** the intermediate layer is based on a polymer from the group of the methacrylate copolymers, preferably being butyl methacrylate-(2-dimethylaminoethyl methacrylate)-methyl methacrylate copolymer or a butyl methacrylate-methyl methacrylate copolymer.

6. Process according to one or more of the preceding claims, **characterized in that** the matrix layer is the pressure-sensitive-adhesive layer facing towards the skin and has a structure based on acrylate copolymers.

7. Process according to claim 6, **characterized in that** the acrylate copolymer having pressure-sensitive-adhesive properties is a carboxylated copolymer, where this is advantageously present in a form neutralized completely or to some extent by one or more basic additives and/or is present in a form crosslinked by aluminium ions or by titanium ions, and the basic additive preferably involves an alkali metal hydroxide, in particular potassium hydroxide, or involves a basic polymer, butyl methacrylate-(2-dimethylaminoethyl methacrylate)-methyl methacrylate copolymer (1:2:1).

8. Process according to Claim 7, **characterized in that** the transdermal system comprises at least one strongly water-binding auxiliary, preferably a carboxylated polymer or its pharmaceutically acceptable salt.

9. Process according to one or more of the preceding claims, **characterized in that** the (meth)acrylate copolymers forming the matrix layers can be processed in solvent-containing systems.

10. Process according to one or more of the preceding claims, **characterized in that** there is at least one other active ingredient and/or auxiliary present which exerts an effect on the central nervous system.

## Revendications

1. Procédé de fabrication d'un système thérapeutique transdermique ayant une teneur en nicotine, constitué :
- d'une couche de fond imperméable à la nicotine,
- d'une couche intermédiaire de matrice contenant de la nicotine et directement contiguë à la couche de fond,
- d'une autre couche de matrice contenant de la nicotine,
- les couches de matrice étant composées de copolymères de (méth-)acrylate, et
- d'une couche de protection pouvant être redétachée, dans lequel l'épaisseur de ces couches ne dépasse pas au total 250 µm et la teneur en nicotine est d'au moins 1,5 mg par cm² de surface d'application,
**caractérisé en ce que**
la couche intermédiaire de matrice contenant de la nicotine n'est pas introduite sur toute la surface par une technique d'impression, de sorte que l'autre couche de matrice forme un bord externe final vis-à-vis de la couche intermédiaire de matrice contenant de la nicotine située à l'intérieur.

2. Procédé selon la revendication 1, **caractérisé en ce que** la résistance à la flexion du système transdermique à appliquer sur la peau ne présente pas une valeur supérieure à 2 cN·cm².

3. Procédé selon la revendication 1 ou 2, **caractérisé en ce que** le système transdermique est exempt de corps d'appui ou de couches de renfort.

4. Procédé selon la revendication 1, 2 ou 3, **caractérisé en ce que** le système transdermique est prévu pour une quantité d'administration de 0,7 à 1,4 mg, de préférence de 1, 0 à 1, 4 mg de nicotine par cm² de peau en l'espace de 24 heures, de préférence pour une durée d'application de 16 heures.

5. Procédé selon l'une quelconque ou plusieurs des revendications précédentes, **caractérisé en ce que** la couche intermédiaire est à base d'un polymère choisi dans le groupe des copolymères de méthacrylate, de préférence le copolymère de méthacrylate de butyle-(méthacrylate de 2-diméthylaminoéthyle)-méthacrylate de méthyle ou un copolymère de méthacrylate de butyle.

6. Procédé selon l'une quelconque ou plusieurs des revendications précédentes, **caractérisé en ce que** la couche de matrice est la couche autoadhésive côté peau et est élaborée à base de copolymères d'acrylate.

7. Procédé selon la revendication 6, **caractérisé en ce que** le copolymère d'acrylate autoadhésif contient des groupements carboxyle, dans lequel celui-ci se présente sous une forme totalement ou partiellement neutralisée par le biais d'un ou de plusieurs additifs basiques et/ou sous une forme réticulée transversalement par des ions d'aluminium ou de titane, et **en ce qu'**il s'agit, dans le cas de l'additif basique, de préférence d'un hydroxyde de métal alcalin, en particulier d'hydroxyde de potassium, ou d'un polymère basique, à savoir un copolymère de méthacrylate de butyle-(méthacrylate de 2-diméthylaminoéthyle)-méthacrylate de méthyle (1:2:1).

8. Procédé selon la revendication 7, **caractérisé en ce que** le système transdermique contient au moins un adjuvant se liant fortement à l'eau, de préférence un polymère contenant des groupements carboxyle ou son sel acceptable au plan pharmaceutique.

9. Procédé selon l'une quelconque ou plusieurs des revendications précédentes, **caractérisé en ce que** les copolymères de (méth-)acrylate constituant les couches de matrice peuvent être transformés en contenant un solvant.

10. Procédé selon l'une quelconque ou plusieurs des revendications précédentes, **caractérisé en ce qu'**il contient au moins un autre principe actif et/ou adjuvant affectant le système nerveux central.
